# EUROPÄISCHE PATENTSCHRIFT

(11) **EP 1 924 298 B1**
(45) Veröffentlichungstag und Bekanntmachung des Hinweises auf die Patenterteilung: **14.07.2010**
(21) Anmeldenummer: 05783006.9
(22) Anmeldetag: 15.09.2005
(51) Int. Cl.: A61L 27/14, A61L 27/28, A61L 27/50, A61L 27/54, A61L 31/08

(54) **BIOVERTRÄGLICHES ANTIMIKROBIELLES NAHTMATERIAL**
BIOCOMPATIBLE ANTIMICROBIAL SUTURE MATERIAL
MATERIAU DE SUTURE ANTIMICROBIEN BIOCOMPATIBLE

(43) Veröffentlichungstag der Anmeldung: 28.05.2008
(73) Patentinhaber: Aesculap AG, 78532 Tuttlingen (DE)
(72) Erfinder: EGGERSTEDT, Sven, 22527 Hamburg (DE); ODERMATT, Erich, K., CH-8200 Schaffhausen (CH); BARGON, Rainer, 88512 Mengen (DE)
(74) Vertreter: Patentanwälte Ruff, Wilhelm, Beier, Dauster & Partner
(86) Internationale Anmeldenummer: PCT/EP2005/009917
(87) Internationale Veröffentlichungsnummer: WO 2007/031101

(56) Entgegenhaltungen:
- EP-A- 1 473 047
- WO-A-02/03899
- WO-A-94/27440

## Beschreibung

Die vorliegende Erfindung betrifft ein bioverträgliches Nahtmaterial mit einer antimikrobiellen Ausrüstung sowie ein Verfahren zur Herstellung des Nahtmaterials und die Bereitstellung des Nahtmaterials für eine Anwendung beim menschlichen oder tierischen Organismus.

Derzeit werden täglich unzählige Eingriffe zu diagnostischen oder therapeutischen Zwecken am menschlichen oder tierischen Körper durchgeführt. Bei diesen Eingriffen werden vielfältigste Implantate, insbesondere Filamentmaterialien, temporär oder dauerhaft in den Körper eingebracht, Untersuchungen mittels mehr oder weniger lange im Körper verweilenden Sonden durchgeführt, die Ver- oder Entsorgung von Stoffen mittels Kathetern gewährleistet sowie vielfältigste Wunden nach medizinischen Eingriffen oder Verletzungen verschlossen. Bei jedem dieser Eingriffe besteht ein erhebliches Risiko der Wundinfektion durch Einschleppung pathogener Keime in den Körper. Insbesondere bei resorbierbaren Filamentmaterialien, beispielsweise für den Wundverschluß, bei denen kein weiterer Eingriff zur Entfernung des resorbierbaren Fremdkörpers beabsichtigt ist, ist es außerordentlich wichtig, dass die Wunde bzw. das Material sowie die Umgebung des Materials bis zur vollständigen Resorption des Materials bzw. zur Heilung der Wunde frei von Infektionen bleibt. Vor allem innerhalb der ersten zwei Wochen nach einem Eingriff besteht ein erhöhtes Risiko von Infektionen durch unerwünschte eingeschleppte Erreger und Vermehrung von pathogenen Keimen aufgrund der geschwächten Immunabwehr und des Wundheilungsprozesses.

Daher wurden bereits viele Anstrengungen unternommen, den Komplikationen durch pathogene Keime vorzubeugen, indem Implantate mit Wirkstoffen ausgerüstet wurden, die die natürliche Immunabwehr unterstützen und die eingeschleppten pathogenen Keime abtöten oder zumindest am Wachstum hindern sollten. So ist beispielsweise aus der US 4,024,872 (US 3,991,766, US 6,528,107) sowie der WO 03/009879 die Imprägnierung bzw. die Zuordnung eines Reservoirs mit einem Breitbandantibiotikum bekannt. Weiterhin beschreiben unter anderem die EP 350147, die US 5,091,442, die WO 03/009879, die US 2002/0193879 sowie die WO 96/22114 die Verwendung von Triclosan in Form von Beschichtungen oder Zusatzstoffen bei der Herstellung von polymeren medizintechnischen Materialien. Die darin beschriebenen medizintechnischen Produkte setzen alle entweder spezifische Antibiotika bzw. Breitbandantibiotika oder den verbreiteten antimikrobiellen Wirkstoff Triclosan zur Bekämpfung von pathogenen Keimen ein. Der Nachteil dieser Wirkstoffe besteht in der allgemein bekannten und mittlerweile in Krankenhäusern sehr gefürchteten Bildung von Resistenzen der pathogenen Mikroorganismen gegen die eingesetzten Antibiotika.

Auch für Triclosan, für welches bisher ein unspezifischer Wirkmechanismus angenommen wurde, ist seit Ende der 90er Jahre ein spezifischer Wirkmechanismus, der in die Fettsäuresynthese der Bakterien eingreift, bekannt geworden. Durch diese Spezifität der Wirkung des Wirkstoffes Triclosan besteht die Gefahr, dass Bakterienstämme bei nicht bakterizid wirksamen Konzentrationen an Triclosan, wie sie vor allem bei der Beschichtung von temporär eingesetzten Produkten wie Kathetern, Handschuhen oder ähnlichem zur Anwendung kommen, Resistenzen entwickeln. Zudem besteht die Gefahr der Ausbildung von Kreuzresistenzen, d.h. dass die Bakterien dadurch auch gegen bestimmte Antibiotika resistent werden können.

In der EP 1473047 A1 wird ein Wundverband auf der Basis von Cellulose mikrobiellen Ursprungs offenbart. Der Wundverband eignet sich vor allem zur Behandlung von chronischen und insbesondere exudierenden Wunden, wie beispielsweise Ulcera. Der Wundverband enthält als antimikrobielles Agens Polyhexamethylenbiguanid (PHMB). Ein weiterer Wundverband mit PHMB als antimikrobielles Agens ist aus der WO 02/03899 A1 bekannt. In der WO 94/27440 A1 werden Antiinfektiva auf der Basis von PHMB mit einem mittleren Molekulargewicht M_{w} von 2900 bis 15 000 beschrieben. Die Antiinfektiva eignen sich dabei insbesondere als Desinfektionsmittel, Wundantiseptika und/oder Wundbehandlungsmittel.

Aufgabe der vorliegenden Erfindung ist es daher, ein bioverträgliches Filamentmaterial mit einer unspezifisch aber dennoch hoch wirksamen antimikrobiellen Ausrüstung zur Verfügung zu stellen, die die Besiedelung durch pathogene Keime effektiv verhindert und gleichzeitig die Resistenzbildung gegen die antimikrobielle Ausrüstung verhindert.

Diese Aufgabe wird gelöst durch ein bioverträgliches Nahtmaterial mit einer antimikrobiellen Ausrüstung, insbesondere in Form einer oberflächlichen Schicht, wobei die Ausrüstung als unspezifisch antimikrobiell wirksame Komponente Polyhexamethylenbiguanid (PHMB) aufweist.

Vorteil des mit PHMB versehenen Nahtmaterials ist der unspezifische Wirkungsmechanismus von PHMB gegenüber pathogenen Keimen, welcher die Gefahr der Resistenzbildung gegen die Wirkstoffkomponente PHMB ausschließt. PHMB ist gut verträglich und wird bei der Schwimmbadwasseraufbereitung eingesetzt, ohne dass unerwünschte Nebenwirkungen auf den menschlichen Organismus bekannt sind. Das erfindungsgemäße Nahtmaterial mit einer antimikrobiellen vorzugsweise oberflächlichen Schicht weist als unspezifisch antimikrobiell wirksame Komponente Polyhexamethylenbiguanid (PHMB) auf.

In einer Ausführungsform kann es erfindungsgemäß vorgesehen sein, daß die antimikrobielle Ausrüstung neben PHMB weitere spezifische oder unspezifische antimikrobielle Komponenten aufweist. Vorzugsweise weist das erfindungsgemäße Nahtmaterial als unspezifisch antimikrobiell wirksame Komponente ausschließlich Polyhexamethylenbiguanid auf.

Das Nahtmaterial liegt vorzugsweise in maximal zweidimensionaler Form vor. Es ist vorzugsweise eindimensional oder zweidimensional ausgebildet.

Bei dem Nahtmaterial kann es sich insbesondere um ein mono- oder multifiles Material handeln, wobei ein multifiles Nahtmaterial bevorzugt ist. Erfindungsgemäß kann es sich bei dem Nahtmaterial um ein Geflecht handeln.

In einer weiteren Ausführungsform der Erfindung ist die unspezifisch antimikrobiell wirksame Komponente PHMB auf der Oberfläche des Nahtmaterials vorhanden. Vorteilhafterweise besitzt das Nahtmaterial eine antimikrobielle Imprägnierung.

In einer weiteren bevorzugten Ausführungsform ist die unspezifisch antimikrobiell wirksame Komponente im Nahtmaterial und vorzugsweise in der äußeren Schicht der Oberfläche vorhanden. Die unspezifisch antimikrobiell wirksame Komponente PHMB ist vorteilhafterweise durch Lösungsmittel in Schichten unter der Oberfläche von vorzugsweise quellbaren Nahtmaterialien einbringbar.

Mit Vorteil ist das PHMB frei von kovalenten Bindungen zum Nahtmaterial. In einer besonderen Ausführungsform kann PHMB ionische Bindungen zu anionischen, d.h. negativ geladenen Materialien ausbilden.

Gemäß einer weiteren Ausbildung der Erfindung ist das PHMB frei verfügbar und ist insbesondere in das umliegende Körpergewebe diffusionsfähig. Je nach Stärke der Bindung zwischen PHMB und Nahtmaterial kann das PHMB durch Auswaschen aus dem Nahtmaterial freisetzbar sein.

Vorzugsweise ist das Nahtmaterial während mindestens 7 Tagen, vorzugsweise während 10 bis 14 Tage, antimikrobiell wirksam.

Weiterhin kann es erfindungsgemäß erwünscht sein, daß es sich bei dem Nahtmaterial um ein bioresorbierbares Material handelt. Dies kann insbesondere dadurch erreicht werden, daß das Nahtmaterial in-vivo hydrolysierbare Polymere aufweist. Mit Vorteil handelt es sich bei den in vivo hydrolysierbaren Polymeren um Polymere auf der Basis von Lactid, Glykolid, Trimethylencarbonat (TMC) und/oder Dioxanon, wobei ein Nahtmaterial aus Lactid- und/oder Glykolid-Polymer, vorzugsweise aus Glykolid-Polymer, besonders bevorzugt ist. Erfindungsgemäß kann es somit vorgesehen sein, daß der Wirkstoff Polyhexamethylenbiguanid (PHMB) nach vollständiger Resorption bzw. nach Resorption der oberflächlichen Schichten des Nahtmaterials jeweils frei verfügbar und ins Gewebe diffusionsfähig ist. Gemäß dieser besonderen Ausführung ist PHMB in seinem Wirkradius nicht auf die Oberfläche des erfindungsgemäßen Nahtmaterials beschränkt, sondern ist auch in der unmittelbaren Umgebung des Nahtmaterials antimikrobiell wirksam.

Gemäß einer anderen Ausführungsform weist das Nahtmaterial einen nicht bioresorbierbaren Stoff auf, insbesondere ein Polymer, vorzugsweise Polyethylen und/oder Polypropylen. Es ist auch möglich, daß es sich bei dem Nahtmaterial um ein teilweise resorbierbares Material handelt.

Vorteilhafterweise beträgt der Gehalt an PHMB auf der Oberfläche des ausgerüsteten Nahtmaterials zwischen 0,1 und 100 µg/cm². Im Falle von im Nahtmaterial vorhandenem PHMB beträgt der Gehalt an PHMB im antimikrobiell ausgerüsteten Nahtmaterial, insbesondere in einem polymeren Nahtmaterial, vorteilhafterweise 2 -3000 ppm.

Gemäß einer weiteren Ausführung weist das Nahtmaterial auf der Oberfläche eine von der antimikrobiellen Ausrüstung verschiedene Beschichtung auf. Die oberflächliche Beschichtung kann resorbierbar oder nicht resorbierbar sein, wobei eine resorbierbare Beschichtung bevorzugt ist. Mit Vorteil weist die resorbierbare Beschichtung ein bioresorbierbares Polymer, insbesondere ein unter in vivo Bedingungen hydrolysierbares Polymer, auf. Bezüglich weiterer Einzelheiten wird auf die obige Beschreibung verwiesen.

Vorteilhaft kann eine Beschichtung zur Erhöhung der Gleitfähigkeit des Nahtmaterials vorgesehen sein, um die Handhabung zu erleichtern. Es ist möglich, daß ein nicht resorbierbares Nahtmaterial eine Beschichtung aus einem resorbierbaren Material aufweist. Durch das resorbierbare Beschichtungsmaterial ist die Abgabe und Diffusion der unspezifisch antimikrobiell wirksamen Komponente PHMB von der Oberfläche des Nahtmaterials in das umliegende Gewebe steuerbar, indem das PHMB erst nach der Resorption des Beschichtungsmaterials wirksam ist.

In einer anderen Ausführungsform handelt es sich bei dem Beschichtungsmaterial um ein nicht resorbierbares Material. Dabei ist die Beschichtung aus dem nicht resorbierbaren Material vorteilhafterweise über die gesamte Fläche der Beschichtung mit Durchtrittsöffnungen zur verzögerten und kontrollierten Abgabe der unspezifisch antimikrobiell wirksamen Komponente PHMB in das Gewebe versehen, um den Kontakt zwischen der unspezifisch antimikrobiell wirksamen Komponente auf oder in dem Nahtmaterial und dem umliegenden Gewebe zu gewährleisten.

Mit Vorteil beträgt der Anteil an Beschichtungsmaterial am Gesamtmaterial (Nahtmaterial einschließlich Beschichtungsmaterial) 0,1 bis 5 Gew.-%, entsprechend 1.000 ppm bis 50.000 ppm.

Bei dem Beschichtungsmaterial handelt es sich vorzugsweise um mindestens ein Material aus der Gruppe umfassend Polyethylen, Polyester, Silikon und Polyurethan. Besonders bevorzugt besteht das Beschichtungsmaterial aus resorbierbaren Materialien enthaltend Lactid, Glykolid, Trimethylencarbonat (TMC), ε-Caprolacton (ECL) oder Dioxanon, die als Monomer oder Polymer vorliegen können.

Gemäß einer anderen Ausgestaltung der Erfindung ist das Nahtmaterial frei von Umhüllungen und die unspezifisch antimikrobiell wirksame Komponente PHMB liegt unmittelbar an der Produktoberfläche vor.

Vorzugsweise sind ca. 60 % des PHMB nach dem Einsatz des Nahtmaterials unmittelbar freisetzbar, wohingegen ca. 40 % des PHMB auch nach 14 Tagen noch im Material gebunden sind und somit das Nahtmaterial direkt an seiner Oberfläche weiterhin eine antimikrobielle Aktivität besitzt.

Gegenstand der Erfindung ist weiterhin ein Verfahren zur Herstellung eines Nahtmaterials gemäß der vorliegenden Erfindung, umfassend die Schritte:
- Herstellung einer Wirkstofflösung aus der unspezifisch antimikrobiell wirksamen Komponente PHMB und einem Lösungsmittel,
- Überführung von PHMB aus der Wirkstofflösung auf und/oder in das Nahtmaterial und
- Trocknung des Nahtmaterials mit dem überführten PHMB.

Bei dem Lösungsmittel kann es sich insbesondere um mindestens ein organisches Lösungsmittel, beispielsweise einen Alkohol, ein Keton oder ein aromatisches Lösungsmittel, handeln. Besonders bevorzugt sind die Lösungsmittel Isopropanol, Ethylacetat, Toluol und/oder Xylol, wobei Ethylacetat besonders bevorzugt ist. In einer weiteren Ausführungsform des erfindungsgemäßen Verfahrens handelt es sich bei dem Lösungsdes erfindungsgemäßen Verfahrens handelt es sich bei dem Lösungsmittel um Wasser. Es ist ebenso möglich, dass Mischungen verschiedener Lösungsmittel, insbesondere der soeben aufgeführten Lösungsmittel, untereinander oder mit Wasser eingesetzt werden.

Die Konzentration des PHMB in der Wirkstofflösung hängt vom Zweck der unspezifisch antimikrobiell wirksamen Komponente ab. Im Falle einer bakteriziden Wirkung ist eine höhere Konzentration als im Falle einer bakteriostatischen Wirkung des PHMBs bevorzugt. Vorzugsweise beträgt die Konzentration des PHMB in der Wirkstofflösung 0,1 bis 20 Gew.-%, insbesondere 0,3 bis 8 Gew.-%.
In einer besonders bevorzugten Ausführungsform wird die Überführung der unspezifisch antimikrobiell wirksamen Komponente PHMB durch Eintauchen des Nahtmaterials in die Wirkstofflösung vorgenommen. Alternativ dazu kann es vorteilhaft sein, die Überführung durch Aufsprühen der Wirkstofflösung auf das Nahtmaterial vorzunehmen.

Bevorzugt handelt es sich bei dem erfindungsgemäßen Verfahren um ein vollautomatisiertes Verfahren, insbesondere um ein vollautomatisiertes Beschichtungsverfahren. So kann das Nahtmaterial ausgehend von einer Abwickelspule vorzugsweise durch eine Tauchöse in einem Tauchbad zwischen zwei sogenannte Abgautschwalzen transportiert werden, deren Anpressdruck variabel eingestellt werden kann. Das Nahtmaterial kann anschließend über mehrere bewegliche Metallrollen geführt und insbesondere mittels IR-Strahlung und Konvektionstrockner getrocknet werden. Vor der Fadenaufwicklung passiert das Nahtmaterial vorzugsweise eine sogenannte Traversiereinheit. Der Fadentransport ist vorteilhafterweise auf eine konstante Fadenspannung während des gesamten Wickelvorgangs ausgelegt.

Das Applikationsverfahren der Wirkstofflösung ist abhängig von der Geometrie und von der Materialbeschaffenheit, insbesondere der Saugfähigkeit des Nahtmaterials. Bei geometrisch anspruchsvollen Raumformen hat es sich als vorteilhaft erwiesen, das Nahtmaterial aus allen Raumrichtungen mit der Wirkstofflösung gegebenenfalls mehrmals zu besprühen, wohingegen bei saugfähigen Nahtmaterialien vorzugsweise ein Tauchverfahren zum Tränken des Nahtmaterials mit der Wirkstofflösung durchgeführt wird.

In einer besonderen Ausgestaltung des Verfahrens wird ein quellbares Nahtmaterial in die Wirkstofflösung getaucht und verbleibt so lange in der Lösung, bis das Nahtmaterial bis zur gewünschten Eindringtiefe gequollen ist. Dabei dringt das PHMB mit dem Lösungsmittel in das Innere des Nahtmaterials ein. Anschließend wird das Lösungsmittel durch Trocknen vorzugsweise bei Raumtemperatur oder gegebenenfalls bei höherer Temperatur und/oder vermindertem Druck entfernt, wobei das PHMB auch nach dem Entfernen des Lösungsmittels in den vormals gequollenen Schichten des Nahtmaterials verbleibt.

Gemäß einer weiteren Ausführungsform des Verfahrens kann es sich bei der Wirkstofflösung auch um eine Suspension oder Emulsion des PHMB in einem geeigneten Suspensions- oder Emulsionsmittel handeln, mit der das erfindungsgemäße Nahtmaterial nach dem oben beschriebenen Verfahren versehen wird.

In einer besonderen Ausführungsform wird die unspezifisch antimikrobiell wirksame Komponente PHMB zusammen mit einem erfindungsgemäßen Beschichtungsmaterial gelöst und anschließend auf das Nahtmaterial aufgebracht. Damit ist die antimikrobielle Ausrüstung wie die Beschichtung des Nahtmaterials in einem Arbeitsschritt möglich und gleichzeitig wird eine Abgabe der unspezifisch antimikrobiell wirksamen Komponente PHMB an das umliegende Gewebe sofort nach dem Einsatz des Nahtmaterials gewährleistet. Für den Fall, daß die Beschichtung nach der unspezifisch antimikrobiell wirksamen Komponente aufgetragen wird, ist es besonders vorteilhaft, wenn das Beschichtungsmaterial resorbierbar ist, so daß nach Resorption der Beschichtung der antimikrobielle Wirkstoff (PHMB) freigesetzt werden kann.

Je nach Applikationsverfahren der Wirkstofflösung kann die Konzentration an Beschichtungsmaterial in der Lösung variieren. Bei einer einmaligen Applikation der Wirkstofflösung wird vorteilhafterweise eine höhere Wirkstoffkonzentration gewählt als bei einer sehr langen Exposition des Nahtmaterials, insbesondere beim Tauchen in die Wirkstofflösung. Mit Vorteil beträgt die Konzentration des Beschichtungsmaterials in der Wirkstofflösung 0,5 bis 5 Gew.-%, vorzugsweise 1 bis 3,5 Gew.-%.

Um eine gute Haftung der unspezifisch antimikrobiell wirksamen Komponente PHMB auf dem Nahtmaterial bzw. ein gutes Eindringen der Wirkstofflösung in das Nahtmaterial zu gewährleisten, kann das Nahtmaterial vor der Auftragung der antimikrobiellen Lösung oberflächenbehandelt werden. Vorzugsweise wird das Nahtmaterial vor der antimikrobiellen Ausrüstung einer Oberflächenbehandlung, insbesondere einem sogenannten Sputtern oder einer Plasmaaktivierung, unterworfen, wobei eine Plasmabehandlung besonders bevorzugt ist. Bevorzugt wird für die Plasmaaktivierung das APPLD- (Atmospheric Pressure Plasma Liquid Deposition) Verfahren verwendet. Durch die Oberflächenvorbehandlung wird die Aufnahmefähigkeit und/oder Haftung, insbesondere der unspezifisch antimikrobiell wirksamen Komponente PHMB, auf der Oberfläche des Nahtmaterials verbessert.
Weiterhin umfasst die Erfindung die Bereitstellung eines Nahtmaterials gemäß der vorliegenden Erfindung für eine Anwendung beim menschlichen oder tierischen Organismus. Das Nahtmaterial kann sowohl innerlich als auch äußerlich angewandt werden. Bevorzugt ist die Bereitstellung für eine innere Anwendung im menschlichen Organismus. Das erfindungsgemäße Nahtmaterial wird vorzugsweise für einen Wundverschluss und/oder bei Hernien verwendet.

### Figurenbeschreibung:

- Figur 1:: Funktionsschaltbild einer Fadenbeschichtungsanlage: Von einer Abwickelspule 1 wird der Faden 2 durch eine Tauchöse 3 in einem Tauchbad 4 zwischen zwei bewegli- chen Abgautschwalzen 5 transportiert, deren Anpreßdruck variabel eingestellt werden kann. Der Faden 2 wird über mehrere bewegliche Metallrollen 6 geführt und mittels IR- Strahler 7 und Konvektionstrockner 8 getrocknet. Vor der Aufwickelspule 10 passiert der Faden eine Traversiereinheit **9**.
- Figur 2:: Rasterelektronische Aufnahme von einem mit PHMB-HCI beschichteten Safil^{®}-Faden
- Figur 3:: Rasterelektronische Aufnahme von einem mit PHMB- Stearat beschichteten Safil^{®}-Faden

Weitere Merkmale der Erfindung ergeben sich durch die nachfolgende Beschreibung von bevorzugten Ausführungsformen in Form von Beispielen in Verbindung mit den Unteransprüchen. Hierbei können die einzelnen Merkmale der Erfindung alleine oder in Kombination miteinander verwirklicht sein.

### Material und Methoden

Nitroprussid (Di-Natriumpentacyanonitrosyl(II)-Dihydrat), Kaliumhexacyanaoferrat (II), Natriumhydroxid, Schwefelsäure (1 M), Wasserstoffperoxid (Perhydrol, 30%), Stearinsäure und Eisen(II)sulfat wurden von Merck KGaA (Darmstadt), Boratpuffer (pH=11) von Riedel-de-Haen (Seelze) und Polyhexamethylenbiguanid (kurz: PHMB) von Arch Chemicals cals GmbH (Ratingen) bezogen. Als Nahtmaterial wurde Safil beige USP 2/0 von B. Braun eingesetzt.

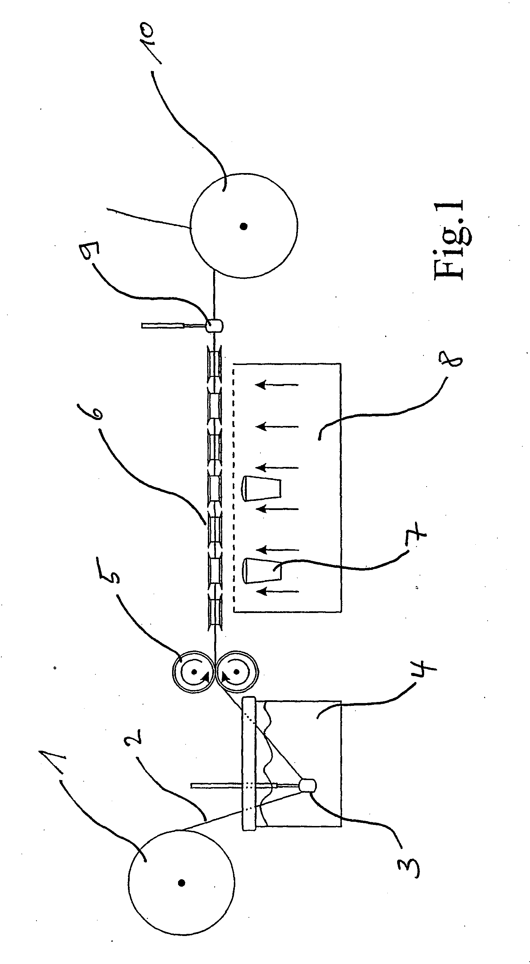

Für die Beschichtungen wurde eine vollautomatische Beschichtungsanlage verwendet (siehe Figur 1).

Die rasterelektronenmikroskopischen Aufnahmen wurden mit einem Zeiss (Oberkochen) Rasterelektronenmikroskop des Typs 435 VP durchgeführt.

Mikrobielle Analysen wurden bei der Firma Confarma AG Schweiz durchgeführt.

### Beispiel 1: PHMB-HCI

### 1.1 Analytik von PHMB-HCI

Die Analytik von PHMB wurde bereits in der Literatur beschrieben (H. Bratt, D. E. Hathway "Characterization of the Urinary Polymer-related Materialform Rats given Poly[biguanidine-1,5-diylhexamethylene hydrochlorid]", Makromol. Chem. 1976, 177, 2591-2606). Die Reagenzlösung wurde wie folgt hergestellt:
10 mL Nitroprussidlösung (10%ig), 10 mL Kaliumhexacyanoferrat(II)-Lösung (10%ig) und 10 mL Natronlauge (10%ig) und 30 mL Wasser wurden gemischt und über Nacht im Kühlschrank gelagert. Die Reagenzlösung kann nicht über mehrere Tage aufbewahrt werden.

### 1.2 Analytik von PHMB-Lösungen

Zur Messung der PHMB-Konzentration in Lösung wurde 1 mL Reagenzlösung mit 5 mL Probe versetzt. Anstelle des Erhitzens auf 45-55 °C, wie in der Literatur beschrieben wird, konnte die Reaktion durch 5 minütiges Beschallen im Ultraschallbad abgeschlossen werden. Die Absorption wurde bei 530 nm mit einem Spektralphotometer bestimmt.

### 1.3 Analytik von PHMB auf Glykolsäurefäden

Zur Analytik des PHMB auf dem Faden muß zuvor die Glykolsäure und der gegebenenfalls im Faden vorhandene Farbstoff D&C violett Nr. 2 als Störkomponenten zerstört werden. Hierzu wurde folgendermaßen verfahren:
6 cm Faden wurden in einen 25-mL-Messkolben gelegt und mit 3 mL Schwefelsäure (1 M) und 3 mL Wasserstoffperoxid (Perhydrol) versetzt. Die Proben werden bei 70 °C im Ultraschallbad für 4 h beschallt und dann für einige Stunden im Bad belassen, bis das Bad die Umgebungstemperatur erreicht hat. Mit Natronlauge wird neutralisiert (pH = 7) und zur Zerstörung überschüssiger Glykolsäure 0,1 mL einer Eisen(II)sulfatLösung (0,1 M) zugesetzt und der Kolben für weitere 4 h im Ultraschallbad beschallt. Die Lösung wird auf 25 mL mittels Boratpuffer (pH=11) aufgefüllt. 1 mL obiger Lösung wurde dann mit 0,2 mL Reagenz und mit 1 mL Boratpuffer versetzt und vermessen.

### 1.4 Beschichtung des Fadens mit PHMB-HCI

In einer Beschichtungsanlage (vgl. auch Figur 1) wurden etwa 100 m eines Safil^{®} beige Fadens (Polyglykolsäure) der Stärke USP·2/0 kontinuierlich beschichtet. Als Beschichtungsmittel wurde eine 20%ige wässrige PHMB-Lösung eingesetzt. Der Anpressdruck der Abgautschwalzen betrug 100 N, die Beschichtungsgeschwindigkeit 5 m/min, die Trocknungstemperatur 350 °C.

Auf dem Faden verblieben gemäß der analytischen Bestimmung aus 5 Messungen 1786 ± 143 mg/m² PHMB-HCI.

### 1.5 Oberflächenuntersuchung

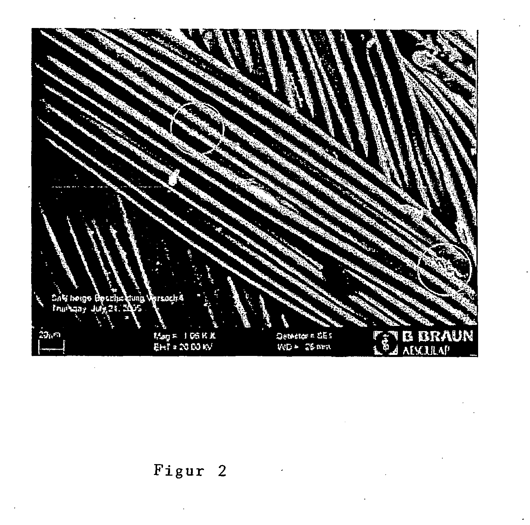

Zur Oberflächenuntersuchung wurden elektronenmikroskopische Aufnahmen durchgeführt. Die Aufnahmen wurden mit einer Beschleunigungsspannung von 20 kV, einem Arbeitsabstand von 25 mm und durch die Detektion von Sekundärelektronen bei 1060 facher Vergrößerung erhalten. Die Kreise deuten Beschichtungsmaterial an (vgl. auch Figur 2).

Die Beschichtung tritt als körniger Belag in Erscheinung (vgl. auch Figur 2). In den Zwischenräumen sind diese deutlich zu erkennen. Die Anlagerung erfolgt relativ gleichmäßig.1.6 Antimikrobielle Wirksamkeit

Eine antimikrobielle Wirksamkeit wurde nach 24 stündiger Einlagerung eines 10 cm langen Fadenstücks der oben erwähnten Probe in 20 mL Phosphat gepufferte Saline (pH=7) nach Inokulation mit 5 x 10⁴ KBE (Kolonien bildende Einheiten) pro Keim zum Zeitpunkt t=0 nachgewiesen. Die Ergebnisse sind in der folgenden Tabelle 1 aufgelistet.

**Tabelle 1. Safil^{®} beige USP 2/0 mit 20%iger PHMB-Lösung beschichtet KMZ = Keimzahl**

| **Zeit (Tage)** | P. aeruginosa ATCC 9027 | S. aureus ATCC 6538 | C. albicans ATCC 10231 | S. epidermidis ATCC 12228 | E. coli ATCC 8739 |
|---|---|---|---|---|---|
| **KMZ (Inokulum)** | 3,50 x 10⁴ | 4,20 x 10⁴ | 3,07 x 10⁴ | 9,75 x 10⁴ | 3,47 x 10⁴ |
| **KMZ nach 0 h** | < 100 | 3,35 x 10⁴ | 3,05 x 10⁴ | 5,00 x 10¹ | 1,50 x 10² |
| **KMZ nach 24 h** | < 10 | < 10 | < 10 | < 10 | < 10 |

Eine effiziente antimikrobielle Wirksamkeit gegen gram-positive und gram-negative Bakterien und gegen einen Blastomyceten wurde nachgewiesen.

### Beispiel 2: PHMB-Stearat

### 2.1. Synthese von PHMB-Stearat

In einer Standardrührapparatur wurden 900 mL Wasser mit 10,8 g Natriumhydroxid-Plätzchen, 88,8 g Stearinsäure verrührt und mit 300 mL PHMB-HCI-Lösung (20%ig) gemischt. Dann wurde die Suspension 2 h bei 80 °C erhitzt, dann im Eisbad auf 4 °C abgekühlt und filtriert. Dreimaliges Resuspendieren in Wasser mit anschließender Filtration und eine Lyophilisation über Nacht lieferte ein amorphes Pulver, welches gemäß IR-spektroskopischer Reinheitsbestimmung frei von Verunreinigungen war.

### 2.2 Beschichtung des Fadens mit PHMB-HCI

In einer Beschichtungsanlage (vgl. auch Figur 1) wurden etwa 100 m eines Safil^{®} beige Fadens der Stärke USP 2/0 kontinuierlich beschichtet. Als Beschichtungsmittel wurde eine 20%ige vollständig gesättigte PHMB-Stearatlösung in Toluol eingesetzt. Der Anpressdruck der Abgautschwalzen betrug 100 N, die Beschichtungsgeschwindigkeit 5 m/min, die Trocknungstemperatur 350 °C. Eine Konzentrationsbestimmung konnte mit der in Beispiel 1 beschriebenen Analytik nicht durchgeführt werden.

### 2.3 Oberflächenuntersuchung

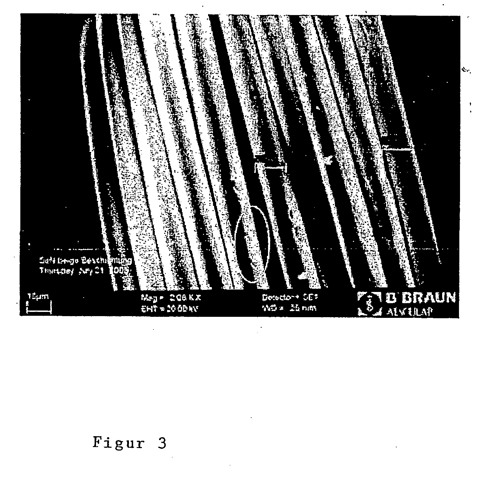

Zur Oberflächenuntersuchung wurden elektronenmikroskopische Aufnahmen durchgeführt. Die Aufnahmen wurden mit einer Beschleunigungsspannung von 20 kV, einem Arbeitsabstand von 25 mm und durch die Detektion von Sekundärelektronen bei 2060 facher Vergrößerung erhalten. Die Kreise in der Abbildung deuten Beschichtungsmaterial an (vgl. auch Figur 3).

Die Beschichtung wirkt einheitlich. Es sind keine Körner und keine Ablagerungen zwischen den Filamenten zu erkennen (vgl. auch Figur 3). Die Anlagerung der Beschichtung ist gleichmäßig.

### 2.4 Antimikrobielle Wirksamkeit

Eine antimikrobielle Wirksamkeit wurde nach 24 stündiger Einlagerung eines 10 cm langen Fadenstücks der oben erwähnten Probe in 20 mL Phosphat gepufferte Saline (pH=7) nach Inokulation mit 5 x 10⁴ KBE (Kolonien bildende Einheiten) pro Keim zum Zeitpunkt t=0 nachgewiesen. Die Ergebnisse sind in der folgenden Tabelle 2 aufgelistet.

**Tabelle 2. Safil^{®} beige USP 2/0 mit gesättigter PHMB-Stearatlösung beschichtet**

| **Zeit (Tage)** | P. aeruginosa ATCC 9027 | S. aureus ATCC 6538 | C. albicans ATCC 10231 | S. epidermidis ATCC 12228 | E. coli ATCC 8739 |
|---|---|---|---|---|---|
| **KMZ (Inokulum)** | 3,50 x 10⁴ | 4,20 x 10⁴ | 3,07 x 10⁴ | 9,75 x 10⁴ | 3,47 x 10⁴ |
| **KMZ nach 0 h** | 7,90 x 10³ | 5,00 x 10⁴ | 3,25 x 10⁴ | 8,10 x 10⁴ | 1,20 x 10⁴ |
| **KMZ nach 24 h** | 1,35 x 10² | 2,65 x 10³ | 2,80 x 10³ | 5,95 x 10³ | 1,70 x 10³ |

Aus Tabelle 2 geht hervor, daß die Beschichtung eine gegenüber bakteriellen Erregern wachstumshemmende Wirkung besitzt.

## Patentansprüche

1. Bioverträgliches Nahtmaterial mit einer antimikrobiellen Ausrüstung, insbesondere in Form einer oberflächlichen Schicht, wobei die Ausrüstung als unspezifisch antimikrobiell wirksame Komponente Polyhexamethylenbiguanid (PHMB) aufweist.

2. Nahtmaterial nach Anspruch 1, **dadurch gekennzeichnet, daß** es mono- oder multifil, insbesondere multifil, ist.

3. Nahtmaterial nach einem der vorhergehenden Ansprüche, **dadurch gekennzeichnet, daß** es als Geflecht vorliegt.

4. Nahtmaterial nach einem der vorhergehenden Ansprüche, **dadurch gekennzeichnet, daß** die unspezifisch antimikrobiell wirksame Komponente PHMB auf der Oberfläche vorliegt.

5. Nahtmaterial nach einem der vorhergehenden Ansprüche, **dadurch gekennzeichnet, daß** es während mindestens 7 Tagen, vorzugsweise während 10 bis 14 Tagen, antimikrobiell wirksam ist.

6. Nahtmaterial nach einem der vorhergehenden Ansprüche, **dadurch gekennzeichnet, dass** es ein resorbierbares, insbesondere hydrolysierbares Polymer, aufweist vorzugsweise aus diesem besteht.

7. Nahtmaterial nach Anspruch 6, **dadurch gekennzeichnet, dass** es sich bei dem Polymer um Lactid- und/oder Glykolid-Polymer handelt, vorzugsweise um Glykolid-Polymer.

8. Nahtmaterial nach einem der vorhergehenden Ansprüche, **dadurch gekennzeichnet, dass** der Gehalt an PHMB auf der Oberfläche des beschichteten Nahtmaterials 0,1 bis 100 µg/cm² beträgt.

9. Nahtmaterial nach einem der vorhergehenden Ansprüche, **dadurch gekennzeichnet, daß** der Gehalt an PHMB im antimikrobiell ausgerüsteten Nahtmaterial 2-3000 ppm beträgt.

10. Nahtmaterial nach einem der vorhergehenden Ansprüche, **dadurch gekennzeichnet, daß** das Nahtmaterial auf der Oberfläche eine von der antimikrobiellen Ausrüstung verschiedene Beschichtung aufweist.

11. Nahtmaterial nach Anspruch 10, **dadurch gekennzeichnet, dass** der Anteil an Beschichtungsmaterial am Gesamtmaterial (Nahtmaterial einschließlich Beschichtungsmaterial) 0,1 - 5 Gew.-% beträgt.

12. Nahtmaterial nach Anspruch 10 oder 11, **dadurch gekennzeichnet, dass** es sich bei dem Beschichtungsmaterial um mindestens eines aus der Gruppe umfassend Polyethlyen, Polyester, Silikon und Polyurethan handelt.

13. Nahtmaterial nach einem der Ansprüche 1 bis 9, **dadurch gekennzeichnet, dass** das Produkt frei von einer Beschichtung ist.

14. Verfahren zur Herstellung eines bioverträglichen Nahtmaterials nach einem der Ansprüche 1 bis 13, umfassend die Schritte:
- Herstellung einer Wirkstofflösung aus der unspezifisch antimikrobiell wirksamen Komponente PHMB und einem Lösungsmittel,
- Überführung von PHMB aus der Wirkstofflösung auf und/oder in das Nahtmaterial und
- Trocknung des Nahtmaterials mit dem überführten PHMB.

15. Verfahren nach Anspruch 14, **dadurch gekennzeichnet, dass** als Lösungsmittel mindestens ein organisches Lösungsmittel, insbesondere ein Alkohol, Keton und/oder ein aromatisches Lösungsmittel, verwendet wird.

16. Verfahren nach einem der Ansprüche 14 oder 15, **dadurch gekennzeichnet, dass** als Lösungsmittel Wasser verwendet wird.

17. Verfahren nach einem der Ansprüche 14 bis 16, **dadurch gekennzeichnet, dass** die Konzentration des PHMB in der Wirkstofflösung 0,1 bis 20 Gew.-%, vorzugsweise 0,3 bis 8 Gew.-%, beträgt.

18. Verfahren nach einem der Ansprüche 14 bis 17, **dadurch gekennzeichnet, dass** die Überführung durch Eintauchen des Nahtmaterials in die Wirkstofflösung vorgenommen wird.

19. Verfahren nach einem der Ansprüche 14 bis 18, **dadurch gekennzeichnet, dass** die Überführung durch Aufsprühen der Wirkstofflösung auf das Nahtmaterial vorgenommen wird.

20. Verfahren nach einem der Ansprüche 14 bis 19, **dadurch gekennzeichnet, dass** zusammen mit der antimikrobiell wirksamen Komponente PHMB ein Beschichtungsmaterial gelöst und anschließend auf das Nahtmaterial gebracht wird.

21. Verfahren nach Anspruch 20, **dadurch gekennzeichnet, dass** die Konzentration des Beschichtungsmaterials in der Wirkstofflösung 0,5 bis 5 Gew.-%, vorzugsweise 1 bis 3,5 Gew.-%, beträgt.

22. Verfahren nach einem der Ansprüche 14 bis 21, **dadurch gekennzeichnet, dass** das Nahtmaterial vor der Auftragung der Wirkstofflösung oberflächenbehandelt wird, insbesondere durch Sputtern oder durch Plasmaaktivierung, insbesondere durch Plasmaaktivierung.

23. Bioverträgliches Nahtmaterial nach einem der Ansprüche 1 bis 13 zur Verwendung beim Wundverschluss und/oder bei Hernien.

## Claims

1. A biocompatible suture material having an antimicrobial finishing, in particular in the form of a surface layer, wherein the finishing includes polyhexamethylene biguanide (PHMB) as a non-specific antimicrobial active component.

2. The suture material according to claim 1, **characterized in that** it is monofil or multifil, in particular multifil.

3. The suture material according to any of the preceding claims, **characterized in that** it is provided as a braid.

4. The suture material according to any of the preceding claims, **characterized in that** the non-specific antimicrobial active component PHMB is present on the surface.

5. The suture material according to any of the preceding claims, **characterized in that** it exhibits antimicrobial activity during at least 7 days, preferably during 10 through 14 days.

6. The suture material according to any of the preceding claims, **characterized in that** it includes an absorbable, in particular hydrolysable polymer, and preferably consists of such a polymer.

7. The suture material according to claim 6, **characterized in that** the polymer is a lactide and/or glycolide polymer, preferably is a glycolide polymer.

8. The suture material according to any of the preceding claims, **characterized in that** the amount of PHMB on the surface of the coated suture material is between 0.1 and 100 µg/cm².

9. The suture material according to any of the preceding claims, **characterized in that** the amount of PHMB in the antimicrobially finished suture material is between 2 and 3000 ppm.

10. The suture material according to any of the preceding claims, **characterized in that** the suture material has on the surface a coating differing from the antimicrobial finishing.

11. The suture material according to claim 10, **characterized in that** a proportion of coating material in the total material (suture material including coating material) is between 0.1 and 5 % by weight.

12. The suture material according to claim 10 or 11, **characterized in that** the coating material is at least one of the group consisting of polyethylene, polyester, silicone and polyurethane.

13. The suture material according to any of the claims 1 through 9, **characterized in that** the product is free of any coating.

14. A method for producing a biocompatible suture material according to any of the claims 1 through 13, comprising the steps:
- preparing an active ingredient solution based on the non-specific antimicrobial active component PHMB and a solvent,
- transferring of PHMB from the active ingredient solution onto and/or into the suture material, and
- drying the suture material having the transferred PHMB.

15. The method according to claim 14, **characterized in that** the solvent used is at least an organic solvent, in particular alcohol, ketone and/or an aromatic solvent.

16. The method according to claim 14 or 15, **characterized in that** the solvent used is water.

17. The method according to any of the claims 14 through 16, **characterized in that** a concentration of PHMB in the active ingredient solution is between 0.1 and 20 % by weight, preferred between 0.3 and 8 % by weight.

18. The method according to any of the claims 14 through 17, **characterized in that** the transferring is performed by immersing the suture material into the active ingredient solution.

19. The method according to any of the claims 14 through 18, **characterized in that** the transferring is performed by spraying the active ingredient solution onto the suture material.

20. The method according to any of the claims 14 through 19, **characterized in that** a coating material is dissolved together with the antimicrobial active component PHMB and subsequently applied to the suture material.

21. The method according to claim 20, **characterized in that** a concentration of coating material in the active ingredient solution is between 0.5 and 5 % by weight, preferred between 1 and 3.5 % by weight.

22. The method according to any of the claims 14 through 21, **characterized in that** the suture material is subject to a surface treatment prior to applying the active ingredient solution, in particular by sputtering or by plasma activation, in particular by plasma activation.

23. A biocompatible suture material according to any of the claims 1 through 13 for use in wound closure and/or with hernias.

## Revendications

1. Matériau de suture biocompatible présentant un apprêt antimicrobien, en particulier sous forme d'une couche superficielle, l'apprêt présentant, comme composant à activité antimicrobienne, non spécifique, du polyhexaméthylènebiguanide (PHMB).

2. Matériau de suture selon la revendication 1, **caractérisé en ce qu'**il s'agit d'un monofil ou d'un multifil, en particulier d'un multifil.

3. Matériau de suture selon l'une quelconque des revendications précédentes, **caractérisé en ce qu'**il se trouve sous forme de tresse.

4. Matériau de suture selon l'une quelconque des revendications précédentes, **caractérisé en ce que** le composant à activité antimicrobienne, non spécifique PHMB se trouve sur la surface.

5. Matériau de suture selon l'une quelconque des revendications précédentes, **caractérisé en ce qu'**il présente une activité antimicrobienne pendant au moins 7 jours, de préférence pendant 10 à 14 jours.

6. Matériau de suture selon l'une quelconque des revendications précédentes, **caractérisé en ce qu'**il présente un polymère résorbable, en particulier hydrolysable et est de préférence constitué par celui-ci.

7. Matériau de suture selon la revendication 6, **caractérisé en ce qu'**il s'agit, pour le polymère, d'un polymère de lactide et/ou de glycolide, de préférence d'un polymère de glycolide.

8. Matériau de suture selon l'une quelconque des revendications précédentes, **caractérisé en ce que** la teneur en PHMB à la surface du matériau de suture revêtu est de 0,1 à 100 µg/cm².

9. Matériau de suture selon l'une quelconque des revendications précédentes, **caractérisé en ce que** la teneur en PHMB dans un matériau de suture apprêté de manière antimicrobienne est de 2-3000 ppm.

10. Matériau de suture selon l'une quelconque des revendications précédentes, **caractérisé en ce que** le matériau de suture présente, à la surface, un revêtement différent de l'apprêt antimicrobien.

11. Matériau de suture selon la revendication 10, **caractérisé en ce que** la proportion de matériau de revêtement par rapport au matériau global (matériau de suture, y compris le matériau de revêtement) est de 0,1-5% en poids.

12. Matériau de suture selon la revendication 10 ou 11, **caractérisé en ce qu'**il s'agit, pour le matériau de revêtement, d'au moins un matériau du groupe comprenant le polyéthylène, le polyester, le silicone et le polyuréthane.

13. Matériau de suture selon l'une quelconque des revendications 1 à 9, **caractérisé en ce que** le produit est exempt d'un revêtement.

14. Procédé pour la production d'un matériau de suture biocompatible selon l'une quelconque des revendications 1 à 13, comprenant les étapes :
- préparation d'une solution de substance active constituée par le composant à activité antimicrobienne, non spécifique PHMB et un solvant,
- transfert du PHMB de la solution de substance active sur et/ou dans le matériau de suture et
- séchage du matériau de suture présentant le PHMB transféré.

15. Procédé selon la revendication 14, **caractérisé en ce qu'**on utilise comme solvant au moins un solvant organique, en particulier un alcool, une cétone et/ou un solvant aromatique.

16. Procédé selon l'une quelconque des revendications 14 ou 15, **caractérisé en ce qu'**on utilise de l'eau comme solvant.

17. Procédé selon l'une quelconque des revendications 14 à 16, **caractérisé en ce que** la concentration en PHMB dans la solution de substance active est de 0,1 à 20% en poids, de préférence de 0,3 à 8% en poids.

18. Procédé selon l'une quelconque des revendications 14 à 17, **caractérisé en ce que** le transfert est réalisé par immersion du matériau de suture dans la solution de substance active.

19. Procédé selon l'une quelconque des revendications 14 à 18, **caractérisé en ce que** le transfert est réalisé par pulvérisation de la solution de substance active sur le matériau de suture.

20. Procédé selon l'une quelconque des revendications 14 à 19, **caractérisé en ce qu'**un matériau de revêtement est dissous ensemble avec le composant à activité antimicrobienne PHMB et ensuite appliqué sur le matériau de suture.

21. Procédé selon la revendication 20, **caractérisé en ce que** la concentration en matériau de revêtement dans la solution de substance active est de 0,5 à 5% en poids, de préférence de 1 à 3,5% en poids.

22. Procédé selon l'une quelconque des revendications 14 à 21, **caractérisé en ce que** le matériau de suture est traité en surface avant l'application de la solution de substance active, en particulier par sputtering ou par activation au plasma, en particulier par activation au plasma.

23. Matériau de suture biocompatible selon l'une quelconque des revendications 1 à 13 destiné à être utilisé lors de la fermeture de plaies et/ou dans le cas de hernies.
